# EUROPEAN PATENT SPECIFICATION

(11) **EP 2 197 823 B1**
(45) Date of publication and mention of the grant of the patent: **14.11.2012**
(21) Application number: 08807761.5
(22) Date of filing: 23.09.2008
(51) Int. Cl.: C07C 35/36, C07C 49/447, C07C 49/637, C07C 69/013, C11B 9/00, C07C 45/62, C07C 45/69

(54) **DECALINE DERIVATIVES AS PERFUMING INGREDIENTS**
DECALINDERIVATE ALS PARFÜMSTOFFE
DÉRIVÉS DE DÉCALINE COMME INGRÉDIENTS POUR PARFUMER

(30) Priority: 03.10.2007 WO PCT/IB2007/054016
(43) Date of publication of application: 23.06.2010
(73) Proprietor: Firmenich S.A., 1211 Geneva 8 (CH)
(72) Inventor: MORETTI, Robert, CH-1212 Grand-lancy (CH); ETTER, Olivier, CH-1225 Chene-bourg (CH)
(74) Representative: Carina, Riccardo Filippo
(86) International application number: PCT/IB2008/053852
(87) International publication number: WO 2009/044310

(56) References cited:
- EP-A- 0 167 709
- EP-A- 1 605 035
- WO-A-2007/010420
- WO-A-2007/031904
- US-A- 4 431 843

## Description

### Technical field

The present invention relates to the field of perfumery. More particularly, it concerns a new class of compounds, α-decalones or decalols with a 6,7,8a-trimethyl substitution, and their use in perfumery to confer odor notes of the citrus type. The present invention concerns also the compositions or articles containing said compounds.

The present invention concerns the use of said compounds in the perfumery industry as well as the compositions or articles containing said compounds.

### Prior art

The compounds of formula (II) as defined herein below are known in the prior art. These compounds have been reported exclusively as chemical intermediates in various papers (e.g. see C. Wilson et al., in Org.&Biomol.Chem. 2003, 2877 or A.L.M. Porto et al., in Tetrahedron Asymmetry, 2006, 1990). However, the prior art does not report or suggest any organoleptic properties of the compounds of formula (11), or any use of said compounds in the field of perfumery.

Some structural analogues known for a perfumery use are reported in the prior art. These disclosures are discussed further below.

### Description of the invention

We have now surprisingly discovered that a compound of formula 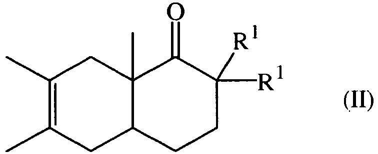 wherein R¹ represents a hydrogen atom or a methyl group; can be used as perfuming ingredient, for instance to impart odor notes of the citrus type, which may have some woody connotation.

At least one R¹ represents a methyl group.

The compounds of formula (II) wherein at least one R¹ is a methyl group are also new compounds, and therefore represent another object of the present invention.

Amongst the invention's compounds, one may cite 2,2,6,7,8a-pentamethyl-3,4,4a,5,8,8a-hexahydro-1(2H)-naphthalenone, which is one of the most appreciated by the perfumer. This compound possesses an odor of the grapefruit and lactonic type, totally devoid of woody aspects. This grapefruit note is very persistent and displays also a coriander aspect as well as an under note reminiscent of cardamom, and an astonishing freshness even at the bottom note.

This velvety-fresh grapefruit note possesses also a nice natural aspect due to the lack of sulfury/perspiralion under notes, which are frequently perceived in other compounds having similar citrusy notes.

The unusual combination of grapefruit and lactonic notes makes 2,2,6,7,8a-pentamethyl-3,4,4a,5,8,8a-hexahydro-1(2H)-naphthalenone a particularly appreciated embodiment of the invention.

Other compounds of formula (II) are also described in Table (I) herein below, together with their odors:

**Table 1 : Structure and odor characteristics of the invention's compounds**

| Structure of compound (II) | Odor |
|---|---|
| 2,6,7,8a-tetramethyl-3,4,4a,5,8,8a-hexahydro-1(2H)-naphthalenone | Citrus/grapefruit, sulfury, vetyver, rooty, nootkatone |

The odor of these compounds distinguishes from the structural analogues disclosed in WO 2007/031904, by having citrus-grapefruit notes, which are absent or not significantly present in the prior art compounds. In fact, the invention's compounds and the prior art analogues have simply a different odor, and said differences lend these two types of compounds to be each suitable for different uses, i.e. to impart different organoleptic impressions.

The invention's compounds distinguish from the ones disclosed in EP 1605035 and having a citrus-grapefruit note, by having a totally different substitution pattern, i.e. not having an alkyl substituent in the position 4 and having a methyl group in the position 7. Therefore, the invention's compounds represent, at least, an alternative to the ones disclosed in EP 1605035 and having a citrus note.

The odor of the invention's compounds distinguishes from the analogues disclosed in EP 47154 by having simply a different odor. In particular, the invention's compounds lack, or do not possess significant, green, herbaceous, peppery, patchouli and/or carrot notes which are characteristic of the prior art compounds, but have citrus/grapefruit notes. Said differences lend the invention's compounds and the prior art compounds to be each suitable for different uses, i.e. to impart different organolpetic impressions.

As mentioned above, the invention concerns the use of a compound of formula (II) as perfuming ingredient. In other words it concerns a method to confer, enhance, improve or modify the odor properties of a perfuming composition or of a perfumed article, which method comprises adding to said composition or article an effective amount of at least a compound of formula (II). By "use of a compound of formula (II)" it has to be understood here also the use of any composition containing compound (II) and which can be advantageously employed in perfumery industry as active ingredients.

Said compositions, which in fact can be advantageously employed as perfuming ingredient, are also an object of the present invention.

Therefore, another object of the present invention is a perfuming composition comprising:
i) as perfuming ingredient, at least one invention's compound as defined above;
ii) at least one ingredient selected from the group consisting of a perfumery carrier and a perfumery base; and
iii) optionally at least one perfumery adjuvant.

By "perfumery carrier" we mean here a material which is practically neutral from a perfumery point of view, i.e. that does not significantly alter the organoleptic properties of perfuming ingredients. Said carrier may be a liquid or a solid.

As liquid carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting example solvents such as dipropyleneglycol, diethyl phthalate, isopropyl myristate, benzyl benzoate, 2-(2-ethoxyethoxy)-1-ethanol or ethyl citrate, which are the most commonly used.

As solid carrier one may cite, as non-limiting examples, absorbing gums or polymers, or yet encapsulating materials. Examples of such materials may comprise wall-forming and plasticizing materials, such as mono, di- or trisaccharides, natural or modified starches, hydrocolloids, cellulose derivatives, polyvinyl acetates, polyvinylalcohols, proteins or pectins, or yet the materials cited in reference texts such as H. Scherz, Hydrokolloids : Stabilisatoren, Dickungs- und Gehermittel in Lebensmittel, Band 2 der Schriftenreihe Lebensmittelchemie, Lebensmittelqualitat, Behr's VerlagGmbH & Co., Hamburg, 1996. The encapsulation is a well known process to a person skilled in the art, and may be performed, for instance, using techniques such as spray-drying, agglomeration or yet extrusion ; or consists of a coating encapsulation, including coacervation and complex coacervation techniques.

By "perfumery base" we mean here a composition comprising at least one perfuming co-ingredient.

Said perfuming co-ingredient is not of the formula (II). Moreover, by "perfuming co-ingredient" it is meant here a compound, which is used in perfuming preparation or composition to impart a hedonic effect. In other words such a co-ingredient, to be considered as being a perfuming one, must be recognized by a person skilled in the art as being able to impart or modify in a positive or pleasant way the odor of a composition, and not just as having an odor.

The nature and type of the perfuming co-ingredients present in the base do not warrant a more detailed description here, which in any case would not be exhaustive, the skilled person being able to select them on the basis of its general knowledge and according to intended use or application and the desired organoleptic effect. In general terms, these perfuming co-ingredients belong to chemical classes as varied as alcohols, aldehydes, ketones, esters, ethers, acetates, nitriles, terpenoids, nitrogenous or sulphurous heterocyclic compounds and essential oils, and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds.

For the compositions which comprise both a perfumery carrier and a perfumery base, other suitable perfumery carrier, than those previously specified, can be also ethanol, water/ethanol mixtures, limonene or other terpenes, isoparaffins such as those known under the trademark Isobar^{®} (origin: Exxon Chemical) or glycol ethers and glycol ether esters such as those known under the trademark Dowanol^{®} (origin: Dow Chemical Company).

By "perfumery adjuvant" we mean here an ingredient capable of imparting additional added benefit such as a color, a particular light resistance, chemical stability, etc. A detailed description of the nature and type of adjuvant commonly used in perfuming bases cannot be exhaustive, but it has to be mentioned that said ingredients are well known to a person skilled in the art.

An invention's composition consisting of at least one compound of formula (II) and at least one perfumery carrier represents a particular embodiment of the invention as well as a perfuming composition comprising at least one compound of formula (II), at least one perfumery carrier, at least one perfumery base, and optionally at least one perfumery adjuvant.

It is useful to mention here that the possibility to have, in the compositions mentioned above, more than one compound of formula (II) is important as it enables the perfumer to prepare accords, perfumes, possessing the odor tonality of various compounds of the invention, creating thus new tools for their work.

Preferably, any mixture resulting directly from a chemical synthesis, e.g. without an adequate purification, in which the compound of the invention would be involved as a starting, intermediate or end-product could not be considered as a perfuming composition according to the invention.

Furthermore, the invention's compound can also be advantageously used in all the fields of modern perfumery to positively impart or modify the odor of a consumer product into which said compound (II) is added. Consequently, a perfumed article comprising: into which said compound (II) is added. Consequently, a perfumed article comprising:
i) as perfuming ingredient, at least one compound of formula (II), as defined above, or an invention's perfuming composition; and
ii) a consumer product base ;
is also an object of the present invention.

For the sake of clarity, it has to be mentioned that, by "consumer product base" we mean here a consumer product, which is compatible with perfuming ingredients. In other words, a perfumed article according to the invention comprises the functional formulation, as well as optionally additional benefit agents, corresponding to a consumer product, e.g. a detergent or an air freshener, and an olfactive effective amount of at least one invention's compound.

The nature and type of the constituents of the consumer product do not warrant a more detailed description here, which in any case would not be exhaustive, the skilled person being able to select them on the basis of its general knowledge and according to the nature and the desired effect of said product.

Examples of suitable consumer product bases include solid or liquid detergents and fabric softeners as well as all the other articles common in perfumery, namely perfumes, colognes or after-shave lotions, perfumed soaps, shower or bath salts, mousses, oils or gels, hygiene products or hair care products such as shampoos, body-care products, deodorants or antiperspirants, air fresheners and also cosmetic preparations. As detergents there are intended applications such as detergent compositions or cleaning products for washing up or for cleaning various surfaces, e.g. intended for textile, dish or hard-surface treatment, whether they are intended for domestic or industrial use. Other perfumed articles are fabric refreshers, ironing waters, papers, wipes or bleaches.

Some of the above-mentioned consumer product bases may represent an aggressive medium for the invention's compound, so that it may be necessary to protect the latter from premature decomposition, for example by encapsulation.

The proportions in which the compounds according to the invention can be incorporated into the various aforementioned articles or compositions vary within a wide range of values. These values are dependent on the nature of the article to be perfumed and on the desired organoleptic effect as well as the nature of the co-ingredients in a given base when the compounds according to the invention are mixed with perfuming co-ingredients, solvents or additives commonly used in the art.

For example, in the case of perfuming compositions, typical concentrations are in the order of 0.001 % to 20 % by weight, or even more, of the compounds of the invention based on the weight of the composition into which they are incorporated. Concentrations lower than these, such as in the order of 0.01% to 15% by weight, can be used when these compounds are incorporated into perfumed articles, percentage being relative to the weight of the article.

The invention's compounds can be prepared according to a method comprising a Diels-Alder reaction between a suitable enone (as dienophile) and 2,3-dimethyl-1,3-butadiene (as diene), and then optionally the reduction of one of the double bonds. This method is further illustrated in the examples herein below.

### Examples

The invention will now be described in further detail by way of the following examples, wherein the abbreviations have the usual meaning in the art, the temperatures are indicated in degrees centigrade (°C) ; the NMR spectral data were recorded in CDCl₃ (if not stated otherwise) with a 360 or 400 MHz machine for ¹H and ¹³C, the chemical displacements δ are indicated in ppm with respect to TMS as standard, the coupling constants J are expressed in Hz.

### Example 1

### Synthesis of compounds of formula (I)

### I) General procedure for the Diels-Alder coupling

In a 500 ml reactor were introduced the AlEtCl₂, or the AlCl₃, 0.1 g of BHT and toluene, or CH₂Cl₂. Then, under vigorous stirring, was added the appropriate cyclohexenone dropwise, so as to maintain the temperature below 30°C. Afterwards was added the diene dropwise and when the reaction ended the reaction mixture was hydrolyzed with 5% aqueous HCl, extracted twice with Et₂O. The organic layer was then washed with a saturated NaHCO₃ aqueous solution, water, brine and then dried over Na₂SO₄. Evaporation of the solvents, chromatography (SiO₂, elution heptane/AcOEt 98:2) and distillation provided the end product.

### II) General procedure for the reduction of the ketone into the alcohol

In a 100 ml flask, maintained under Ar atmosphere, were introduced 2 molar equivalents, with respect of the ketone, of LiAlH₄ in Et₂O. Then the appropriate naphthalenone was added dropwise, so as to maintain the reflux. After completion of the reaction the mixture was stirred for 30 minutes at reflux. Afterwards the reaction mixture was hydrolyzed with a stoechiometric amount of aqueous NaOH and the organic layer was dried over Na₂SO₄. Evaporation of the solvents and distillation provided the end product.

### III) General procedure for the hydrogenation of the naphthalenone into the perhydro naphthalenone

In a 100 ml flask were introduced the appropriate naphthalenone, ethyl acetate and 10% w/w, relative to the naphthalenone, of Pd/C 5%. The mixture was thus stirred under H₂, at a room temperature, until consumption of the theoretical amount of hydrogen. Afterwards, the reaction mixture was filtered over Nylon 6/6. Evaporation of the solvents and distillation provided the end product.

### IV) General procedure for the esterification of the alcohol

In a 250 ml flask were introduced the appropriate alcohol, CH₂Cl₂, dimethylaminopyridine, pyridine and the appropriate carboxylic anhydride. The mixture was thus stirred for 24 hours at room temperature. When the reaction finished the reaction mixture was hydrolyzed with 5% aqueous HCl, extracted twice with Et₂O. The organic layer was then washed with an aqueous solution of CuSO₄, a saturated NaHCO₃ aqueous solution, water, brine and then dried over Na₂SO₄. Evaporation of the solvents provided the end product.

### 2,2,6,7,8a-pentamethyl-3,4,4a,5,8,8a-hexahydro-1(2H)-naphthalenone

Prepared according to general procedure I), with the following quantities:
2,6,6-Trimethyl-2-cyclohexen-1-one (27.6 g; 0.2 mol)
Ethyl aluminium dichloride (1 molar solution in hexanes; 40 ml; 0.04 mol)
Dimethylbutadiene (24.7 g; 0.3 mol), Dichloromethane (200 ml)
The title compound was obtained in 72 % yield (1 isomer).
B.p. = 86 °C/0.025 mbar
¹H-NMR: 1.08 (s, 3 H); 1.12 (s, 3 H); 1.20 (s, 3 H); 1-44-1.84 (m, 7 H); 1.60 (broad s, 3 H); 1.62 (broad s, 3 H); 2.22-2.40 (m, 2H).
¹³C-NMR: 219.61 (s); 122.40 (s); 121.78 (s); 47.78 (s); 43.34 (s); 40.06 (d); 38.25 (t); 37.83 (t); 35.55 (t); 29.39 (q); 28.72 (q); 25.12 (t); 22.00 (q); 19.01 (q); 18.96 (q).

### 2,6,7, 8a-tetramethyl-3,4,4a,5,8,8a-hexahydro-1(2H)-naphthalenone

Prepared according to general procedure I), with the following quantities:
2,6-Dimethyl-2-cyclohexen-1-one (17.6 g; 0.1 mol)
Ethyl aluminium dichloride (1 molar solution in hexanes; 50 ml; 0.05 mol)
Dimethylbutadiene (16.4 g; 0.2 mol), Dichloromethane (200 ml)
The title compound was obtained in 55 % yield as a 16:84 mixture of isomers.
B.p. = 80 °C/0.021 mbar
1H-NMR (major isomer): 1.00 (d, J = 7 Hz, 3 H); 1.05 (s, 3 H); 1.32 (m, 1 H); 1.42-1.87 (m, 5 H); 1.61 (broad s, 3 H); 1.63 (broad s, 3 H); 2.03 (m, 1 H); 2.27 (m, 1 H); 2.44 (m, 1 H); 2.73-2.83 (m, 1 H).

### Example 2

### Preparation of a perfuming composition

A feminine perfume of the floral, citrus-musk type was prepared by admixing the following ingredients:

| Ingredient | Parts by weight |
|---|---|
| Benzyl acetate | 20 |
| 10%* Cis-3-Hexenol acetate | 40 |
| 10%* Acetophenone | 40 |
| C 10 Aldehyde | 10 |
| 10%* Aldehyde C 12 | 30 |
| Aldehyde C 8 | 10 |
| 10%* Aldehyde C 9 | 20 |
| 10%* Nonalactone gamma | 10 |
| 10%* Ethyl butyrate | 20 |
| Allyl caproate | 20 |
| 10%* Laevo-Carvone | 25 |
| Citral | 5 |
| 4-Cyclohexyl-2-methyl-2-butanol | 100 |
| Allyl cyclohexylpropionate | 10 |
| Decalactone gamma | 10 |
| 10%* Dorinone^{® 1)} Beta | 40 |
| Exaltolide^{®2)} Total | 20 |
| Geraniol | 20 |
| Phenylethyl isobutyrate | 10 |
| Limette | 130 |
| Linalol | 400 |
| Dodecenal | 50 |
| 10%* 4-(2,2,C-3,T-6-tetramethyl-R-1-cyclohexyl)-3-buten-2-one | 40 |
| Hedione^{®3)} | 250 |
| 10%* Cis-3-Hexenol | 40 |
| 0.01%* Pyrazobutyl⁴⁾ | 10 |
| 0.01%* 8-Mercapto-3-P-menthanone | 40 |
| Orange terpenes | 1000 |
| Terpineol | 30 |
| Ionone Beta | 25 |
| 2,4-Dimethyl-3-cyclohexen-1-carboxaldehyde | 25 |
| | 2500 |

| | |
|---|---|
| * in dipropyleneglycol 1) 1-(2,6,6-Trimethyl-1-cyclohexen-1-yl)-2-buten-1-one; origin: Firmenich SA, Geneva, CH 2) 15-Pentadecanolide; origin: Firmenich SA, Geneva, CH 3) Methyl dihydrojasmonate; origin: Firmenich SA, Geneva, CH 4) origin: Firmenich SA, Geneva, CH | |

The addition of 100 parts by weight of 2,2,6,7,8a-pentamethyl-3,4,4a,5,8,8a-hexahydro-1(2H)-naphthalenone to the above-described perfuming composition reinforced in a very noticeable manner the citrus connotation of the perfume by providing a sparkling grapefruit aspect.
Moreover, the impact of the citrus notes is strongly increased over time, rendering this note perceivable over at least 24 hour on a smell strip, an astonishing performance for such a note.
Such effect was totally absent when any one of the above-mentioned prior art structural analogues was used instead of the invention's compound.
When prior art compounds such as 6,6-dimethoxy-2,5,5-trimethyl-2-hexene or 4,7-dimethyl-6-octen-3-one, known for imparting citrus notes, were used, the effect was quite different and much weaker.

### Example 3

### Preparation of a perfuming composition

A perfuming base for a shower gel, of the citrus-grapefruit-Yuzu type, was prepared by admixing the following ingredients:

| Ingredient | Parts by weight |
|---|---|
| 10%* Ambrox^{® 1)} | 50 |
| 1%* Ethyl 2-methylpentanoate | 25 |
| 10%* Undecalactone gamma | 20 |
| Citronellol | 40 |
| 4-Cyclohexyl-2-methyl-2-butanol | 250 |
| 1,1-Dimethyl-2-phenylethyl butanoate | 35 |
| Coumarine | 20 |
| Damascenone ²⁾ | 5 |
| 10%* Damascone Alpha³⁾ | 20 |
| Decalactone gamma | 5 |
| Exaltenone^{® 4)} | 30 |
| Exaltolide^{® 5)} Total | 580 |
| 10%* 7-Methyl-2H,4H-1,5-benzodioxepin-3-one | 15 |
| 3-(3-Isopropyl-1-phenyl)butanal | 5 |
| Florol^{® 6)} | 170 |
| 3-(4-Methoxyphenyl)-2-methylpropanal | 120 |
| Geraniol | 10 |
| Habanolide^{® 7)} | 270 |
| Hydroxycitronellal | 110 |
| Iso E^{® 8)} Super | 700 |
| Phenoxy isobutyrate | 20 |
| (Z)-3-Hexenyl methyl carbonate | 10 |
| Lilial^{® 9)} | 300 |
| Lyral^{® 10)} | 100 |
| Mandarine essential oil | 60 |
| Muscenone¹¹⁾ | 40 |
| Hedione® ¹²⁾ | 500 |
| 1%* Rose oxide | 25 |
| Polysantol^{® 13)} | 15 |
| Orange essential oil | 65 |
| Romandolide^{® 14)} | 285 |
| Cis-3-Hexenyl salicylate | 70 |
| Vanilline | 30 |
| | 4000 |

| | |
|---|---|
| * in dipropyleneglycol 1) 8,12-Epoxy-13,14,15,16-tetranorlabdane; origin: Firmenich SA, Geneva, CH 2) (E)-1-(2,6,6-Trimethyl-1,3-cyclohexadien-1-yl)-2-buten-1-one; origin: Firmenich SA, Geneva, CH 3) (E)-1-(2,6,6- Trimethyl-2,4-cyclohexadien-1-YL)-2-buten-1-one; origin: Firmenich SA, Geneva, CH 4) 4/5-Cyclopentadecen-1-one; origin: Firmenich SA, Geneva, CH 5) 15-Pentadecanolide; origin: Firmenich SA, Geneva, CH 6) Tetrahydro-2-isobutyl-4-methyl-4(2H)-pyranol; origin: Firmenich SA, Geneva, CH 7) Pentadecenolide; origin: Firmenich SA, Geneva, CH 8) 1-(Octahydro-2,3,8,8-tetramethyl-2-naphtalenyl)-1-ethanone; origin: International Flavors & Fragrances, USA 9) 3-(4-Tert-butylphenyl)-2-methylpropanal; origin: Givaudan-Roure SA, Vernier, CH 10) 4/3-(4-hydroxy-4-methylpentyl)-3-cyclohexene-1-carbaldehyde; origin: International Flavors & Fragrances, USA 11) 3-Methyl-(4/5)-cyclopentadecenone; origin: Firmenich SA, Geneva, CH 12) Methyl dihydrojasmonate; origin: Firmenich SA, Geneva, CH 13) (1'R,E)-3,3-Dimethyl-5-(2',2',3'-trimethyl-3'-cyclopenten-1'-yl)-4-penten-2-ol; origin: Firmenich SA, Geneva, CH 14) [1-(3',3'-Dimethyl-1'-cyclohexyl)ethoxycarbonyl]methyl propanoate; origin: Firmenich SA, Geneva, CH | |

The addition of 800 parts by weight of 2,2,6,7,8a-pentamethyl-3,4,4a,5,8,8a-hexahydro-1(2H)-naphthalenone to the above-described perfuming composition imparted a clear connotation of the grape-fruit/yuzu type, providing thus to the perfuming composition volume, freshness and tenacity.

## Claims

1. A perfuming composition comprising
i) at least one compound of formula 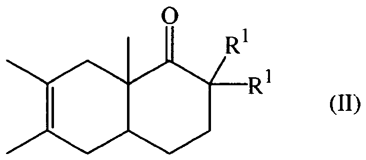 wherein R¹ represents a hydrogen atom or a methyl group, and at least one R¹ represents a methyl group
ii) at least one ingredient selected from the group consisting of a perfumery carrier and a perfumery base; and
iii) optionally at least one perfumery adjuvant.

2. A perfuming composition according to claim 1, **characterized in that** said compound (II) is 2,2,6,7,8a-pentamethyl-3,4,4a,5,8,8a-hexahydro-1(2H)-naphthalenone.

3. A compound of formula 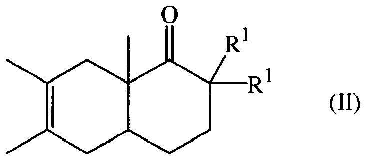 wherein R¹ represents a hydrogen atom or a methyl group, and at least one R¹ represents a methyl group.

4. A compound according to claim 3, 2,2,6,7,8a-pentamethyl-3,4,4a,5,8,8a-hexahydro-1(2H)-naphthalenone.

5. Use as perfuming ingredient of a compound of formula (I), as defined in claim 1 or 2.

6. A perfumed article comprising:
i) at least one compound of formula (II), as defined in claim lor 2; and
ii) a consumer product base.

7. A perfumed article according to claim 6, **characterized in that** the consumer product base is a solid or liquid detergent, a fabric softener, a perfume, a cologne or after-shave lotion, a perfumed soap, a shower or bath salt, mousse, oil or gel, a hygiene product, a hair care product, a shampoo, a body-care product, a deodorant or antiperspirant, an air freshener, a cosmetic preparation, a fabric refresher, an ironing water, a paper, a wipe or a bleach:

## Patentansprüche

1. Parfümierende Zusammensetzung, umfassend
i) mindestens eine Verbindung der Formel 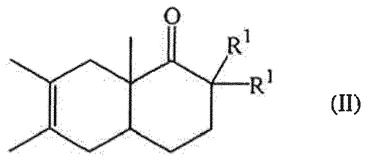 wobei R¹ ein Wasserstoffatom oder eine Methylgruppe darstellt und mindestens ein R¹ eine Methylgruppe darstellt;
ii) mindestens einen Bestandteil, ausgewählt aus der Gruppe, bestehend aus einem Träger für Parfümfabrikation und einer Basis für Parfümfabrikation; und
iii) gegebenenfalls mindestens einen Hilfsstoff für Parfümfabrikation.

2. Parfümierende Zusammensetzung gemäß Anspruch 1, **dadurch gekennzeichnet, dass** die Verbindung (II) 2,2,6,7,8a-Pentamethyl-3,4,4a,5,8,8a-hexahydro-1(2H)-naphthalinon ist.

3. Verbindung der Formel 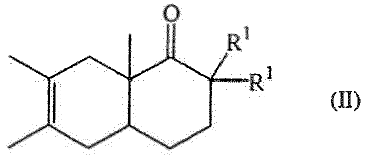 wobei R¹ ein Wasserstoffatom oder eine Methylgruppe darstellt und mindestens ein R¹ eine Methylgruppe darstellt.

4. Verbindung gemäß Anspruch 3, 2,2,6,7,8a-Pentamethyl-3,4,4a,5,8,8a-hexahydro-1(2H)-naphthalinon.

5. Verwendung als parfümierender Bestandteil einer Verbindung der Formel (II), wie definiert in Anspruch 1 oder 2.

6. Parfümierter Gegenstand, umfassend:
i) mindestens eine Verbindung der Formel (II), wie definiert in Anspruch 1 oder 2; und
ii) eine Verbraucherproduktbasis.

7. Parfümierter Gegenstand gemäß Anspruch 6, **dadurch gekennzeichnet, dass** die Verbraucherproduktbasis ein festes oder flüssiges Detergens, ein Weichspüler, ein Parfüm, ein Kölnischwasser oder eine Aftershavelotion, eine parfümierte Seife, ein Dusch- oder Badesalz, -schaum, -öl oder -gel, ein Hygieneprodukt, ein Haarpflegeprodukt, ein Shampoo, ein Körperpflegeprodukt, ein Deodorant oder Antiperspirant, ein Luftverbesserer, eine kosmetische Zubereitung, ein Gewebeauffrischer, ein Bügelwasser, ein Papier, ein Wischtuch oder ein Bleichmittel ist.

## Revendications

1. Composition parfumante comprenant
i) au moins un composé de formule 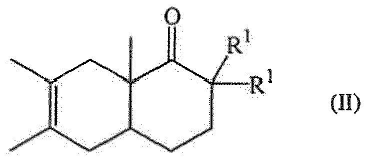 dans laquelle R¹ représente un atome d'hydrogène ou un groupe méthyle, et au moins un R¹ représente un groupe méthyle;
ii) au moins un ingrédient choisi dans le groupe constitué d'un véhicule de parfumerie et d'une base de parfumerie; et
iii) éventuellement, au moins un adjuvant d'usage courant en parfumerie.

2. Composition parfumante selon la revendication 1, **caractérisée en ce que** ledit composé (II) est la 2,2,6,7,8a-pentaméthyl-3,4,4a,5,8,8a-hexahydro-1(2H)-naphtalénone.

3. Composé de formule 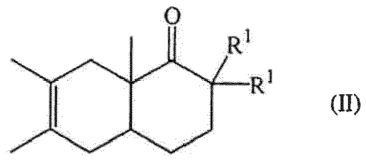 dans laquelle R¹ représente un atome d'hydrogène ou un groupe méthyle, et au moins un R¹ représente un groupe méthyle.

4. Composé selon la revendication 3, la 2,2,6,7,8a-pentaméthyl-3,4,4a,5,8,8a-hexahydro-1 (2H)-naphtalénone.

5. Utilisation, en tant qu'ingrédient parfumant, d'un composé de formule (II), tel que défini dans la revendication 1 ou 2.

6. Article parfumé comprenant:
i) au moins un composé de formule (II), tel que défini dans la revendication 1 ou 2; et
ii) une base de produit de consommation.

7. Article parfumé selon la revendication 6, **caractérisé en ce que** la base de produit de consommation est un détergent solide ou liquide, un adoucissant pour textile, un parfum, une eau de Cologne ou une lotion après-rasage, savon parfumé, un sel, une mousse, une huile ou un gel de bain ou de douche, un produit d'hygiène, un produit de soin capillaire, un shampooing, un produit de soin corporel, un déodorant ou un anti-transpirant, un désodorisant d'air ambiant, une préparation cosmétique, une eau de linge, une eau de repassage, un papier, une lingette ou un agent de blanchiment.
